# EUROPEAN PATENT SPECIFICATION

(11) **EP 1 368 082 B1**
(45) Date of publication and mention of the grant of the patent: **04.06.2008**
(21) Application number: 02750573.4
(22) Date of filing: 06.03.2002
(51) Int. Cl.: A61M 5/32

(54) **Safety shield for medical needles**
Schutzvorrichtung für medizinische Nadeln
Protecteur d'aiguille médicale

(30) Priority: 14.03.2001 US 275886 P; 14.03.2001 US 275810 P; 08.06.2001 US 296968 P; 27.06.2001 US 892593
(43) Date of publication of application: 10.12.2003
(73) Proprietor: Tyco Healthcare Group LP, Mansfield, MA 02048 (US)
(72) Inventor: FERGUSON, F., Mark, Salt Lake City, UT 84124 (US); FISER, Richard, Kirkwood, St. Louis Co., MO 63122 (US); CURTIS, James, R., Deland, FL 32724 (US); OWEN, Charles, V., Highland, UT 84003 (US); THORNE, David, L., Kaysville, UT 84037 (US); NELSON, Mark, A., Sandy, UT 84093 (US); BARRUS, Roy, L., Bountiful, UT 84087 (US); THORNE, Gale, H., Jr., Bountiful, UT 84010 (US); WEILBACHER, Eugene, E., Chesterfield, MO 63005 (US); THORNE, Michael, Bountiful, UT 84010 (US); BROWN, Steven, Roy, UT 84067 (US); SOLOMON, Donald, Ogden, UT 84403 (US)
(74) Representative: Tomlinson, Edward James
(86) International application number: PCT/US2002/006784
(87) International publication number: WO 2002/072181

(56) References cited:
- EP-A- 0 713 710
- EP-A- 0 807 443
- EP-A- 1 116 493
- WO-A-00/16832
- WO-A-00/38765
- WO-A-01/32241
- WO-A-93/17732
- WO-A-97/31666
- GB-A- 2 369 779
- US-A- 4 735 618
- US-A- 4 804 372
- US-A- 4 911 694
- US-A- 5 246 427
- US-A- 5 256 152
- US-A- 5 490 841
- US-A- 6 135 530
- US-B1- 6 171 284

## Description

### FIELD OF THE INVENTION

This invention relates generally to safety shields for medical needles, and more particularly, to safety shields that are extensible to shield a needle point of a medical needle.

### BACKGROUND OF THE INVENTION

Problems associated with inadvertent needle sticks are well known in the art of blood sampling, percutaneous medication injection and other medical procedures involving use of medical needles. Significant attention has been focused on needle stick problems due to the contemporary sensitivity of exposure to AIDS, Hepatitis and other serious blood-borne pathogen exposures.

Procedures for removing a needle from a patient commonly require a technician to use one hand to place pressure at the wound site where the needle is being withdrawn, while removing the needle device with the other hand. It is also common practice for an attending technician to give higher priority to care for the wound than is given to disposal of a needle. In the case of typical needle devices without safety shields, such priority either requires the convenience of an available sharps container within reach or another means for safe disposal without leaving the patient's side. Providing adequate care while following safety procedures is often compounded by the patient's physical condition and mental state, such as in burn units and psychiatric wards. Under such condition, it is difficult to properly dispose of a used needle while caring for a patient.

Examples of known needle safety shields are described, for example, in the documents US 4,911,694, US 5,246,427, US 6,171,284, WO 97/31666 and EP 0 713 710 A1.

The widespread knowledge and history associated with needle care and disposal problems have resulted in numerous devices for preventing accidental needle sticks. Problems of current safety devices include difficulty of use and high cost due to their complexity and number of parts.

There remains a need to provide a more satisfactory solution to a needle safety device.

### SUMMARY OF THE INVENTION

The present invention was developed to fill a need for a device which effectively and inexpensively protects a medical needle after use. The present invention seeks to resolve a number of the problems which have been experienced in the background art. More specifically, the apparatus of this invention constitute an important advance in the art of safety needle devices.

In one particular embodiment, a medical needle shield apparatus is provided, in accordance with the principles of the present disclosure. The medical needle shield apparatus includes a needle hub having a collar and a shield having a proximal end receivable by the collar. The shield being extensible from a retracted position to an extended position. The interior cavity defines notches that receive tabs formed with the proximal end of the shield to lock the needle hub with the shield. The collar can be monolithically formed with the needle hub. The needle hub can have a luer fitting configured to attach to a syringe.

The medical needle shield apparatus includes a needle hub having a collar defining an interior cavity. The needle hub supports a needle having a distal end. A needle shield component includes a proximal and distal end. The proximal end of the shield being receivable within the interior cavity of the collar in an interlocking engagement. The shield being extensible from a retracted position to an extended position wherein the distal end of the shield encloses at least a portion of the distal end of the needle. The shield can include two or more hingedly connected segments, for instance, the shield may include four hingedly connected segments. The tabs may be biased for receipt within the notches.

The shield may be locked in the extended position. The shield may also be irreversibly locked in the extended position. The shield may be locked in the extended position via engagement with the needle or through locking engagement of two or more adjacent hingedly connected shield segments. The shield can include a lock that engages the needle to lock the shield in the extended position. The lock may include a portion configured to flexibly engage the needle and bias to lockably retain the needle. Alternatively, the shield includes locking means to lock the shield in the extended position.

In another embodiment, the distal end of the shield includes a linear bearing configured to enclose at least a portion of the distal end of the needle. The linear bearing may be hingedly connected to and disposed within the distal end of the shield. The linear bearing can be configured to slide along the needle during extension of the shield. The linear bearing can have many configurations such as duckbill or full cylinder. The linear bearing may have a flap configured to align the linear bearing with the needle.

In another embodiment, the shield includes a proximal segment engaging a retention catch or stop formed with the proximal end of the shield to releasably dispose the shield in the retracted position. The needle hub may include a stop or catch which engages the shield in the extended position. The medical needle shield apparatus may include a sheath engageable with the needle hub. The sheath can have guide rails configured to facilitate engagement of the sheath and the needle hub.

In another alternate embodiment, the shield has an articulating actuator configured to urge the shield towards the extended position. The medical needle shield apparatus may further include a tape down member attached to the shield and configured to facilitate extension of the shield. The needle hub can include guide surfaces to facilitate engagement of the shield and the needle hub. The needle hub may include at least one catch or protrusion and the shield may include at least one corresponding protrusion or catch which engage to lock the shield in the extended position.

The shield segments may be connected via living hinges. The segments can include relief portions formed adjacent the living hinges. The relief portions can be configured to flex inwardly toward the needle. The shield may have a proximal segment including at least one rib. The at least one rib may have a transverse orientation.

The medical needle shield apparatus may be configured for use with a port access needle. A pair of wings may be attached to the proximal end of the shield. The shield can include a needle latch that engages the needle in the extended position.

In yet another alternate embodiment, the lock mechanism includes at least one catch for engagement with a corresponding protrusion disposed on the shield in the extended position. The catch may include a capture hole, recess or indentation. The catch may also include a flanged surface. Alternatively, the lock mechanism can include at least one catch for engagement with a corresponding protrusion disposed on the hub in the extended position. The lock may include at least one protrusion or catch for engagement with a corresponding catch or protrusion disposed on the shield in the extended position.

In another embodiment, the medical needle shield apparatus includes a latch which secures a distal segment of the shield in the extended position. The distal segment has an underside including a surface extending over at least a portion of the distal segment for retaining the distal end of the needle. The latch may include at least one lock associated with the distal segment for securing the distal segment to the shield in the extended position. The medical needle shield apparatus may include a retainer for holding the segments in a retracted position. The retainer can include a retainer arm disposed on the needle hub and extending to a corresponding catch disposed on the shield in the retracted position.

In another embodiment, at least one segment includes at least one needle guide for facilitating extension of the segments when extending the shield over the needle. The shield may further include a raised surface for aid in urging the shield to the extended position.

In another embodiment, the medical needle shield apparatus includes a needle hub including a collar and a shield having a distal end and a proximal end receivable by the collar. The shield being extensible from a retracted position to an extended position, wherein the shield includes at least one catch and at least one corresponding protrusion which engage to lock the shield in the extended position. The protrusion can include a latching arm extending from a segment and the catch including a flanged surface disposed adjacent a hinged connection.

In yet another embodiment, the medical needle shield apparatus includes an extensible shield having at least two hingedly connected segments, wherein the segments include reliefs formed adjacent the hinges and configured to flex inwardly.

### BRIEF DESCRIPTION OF THE DRAWINGS

The foregoing and other features and advantages of the present invention will be more fully understood from the following detailed description of the illustrative embodiments, taken in conjunction with the accompanying drawings in which:
FIGURE 1 is a perspective view of a medical needle safety shield apparatus in a retracted position, in accordance with the principles of the present invention;
FIGURE 2 is a part cross-sectional view of the safety shield apparatus shown in FIGURE 1;
FIGURE 3 is a perspective view of the safety shield apparatus illustrated in FIGURE 1 at mid-extension;
FIGURE 4 is a perspective view of the safety shield apparatus illustrated in FIGURE 1 fully extended;
FIGURE 5 is a cross-sectional view of the safety shield apparatus shown in FIGURE 4;
FIGURE 6 is a perspective view of a hub of the safety shield apparatus illustrated in FIGURE 1;
FIGURE 7 is a perspective view of a shield separate from the hub of the safety shield apparatus illustrated in FIGURE 1;
FIGURE 8 is a side view of the safety shield apparatus illustrated in FIGURE 1;
FIGURE 9 is a perspective view of an alternate embodiment of the safety shield apparatus;
FIGURE 10 is a cross-sectional view of the safety shield apparatus illustrated in FIGURE 1 having an articulating button;
FIGURE 11 is a cross-sectional view of the safety shield apparatus illustrated in FIGURE 10;
FIGURE 12 is a perspective view of the safety shield apparatus illustrated in FIGURE 1 showing an alternate embodiment of a linear bearing;
FIGURE 13 is a perspective view of an underside of the safety shield apparatus illustrated in FIGURE 9 showing a barbed flap lock;
FIGURE 14 is a cutaway view of an alternative embodiment of a flap lock securing a medical needle of the safety shield apparatus illustrated in FIGURE 13;
FIGURE 15 is a cutaway view of an alternate embodiment of the lock of the safety shield apparatus illustrated in FIGURE 13;
FIGURE 15A is a plan view of operation of a rectangular lock;
FIGURE 16 is a cutaway view of an alternate embodiment of the lock of the safety shield apparatus illustrated in FIGURE 13;
FIGURE 17 is a view of the underside of the safety shield apparatus illustrated in FIGURE 7;
FIGURE 17A is a partial cross-sectional view of a hinge component of the safety shield apparatus in accordance with the present disclosure;
FIGURE 17B is a cutaway plan view of the hinge component of FIGURE 17 without the application of stressing forces;
FIGURE 17C is a cutaway plan view of the hinge component of FIGURE 17 with the application of stressing forces;
FIGURE 18 is a perspective view of an alternate embodiment of the safety shield apparatus;
FIGURE 19 is a cross-sectional view of an alternate embodiment the safety shield apparatus;
FIGURE 20 is a perspective view of the safety shield apparatus illustrated in FIGURE 1 with a sheath;
FIGURE 21 is a rear perspective view, in part cross-section, of the sheath shown in FIGURE 20;

### DETAILED DESCRIPTION OF THE INVENTION

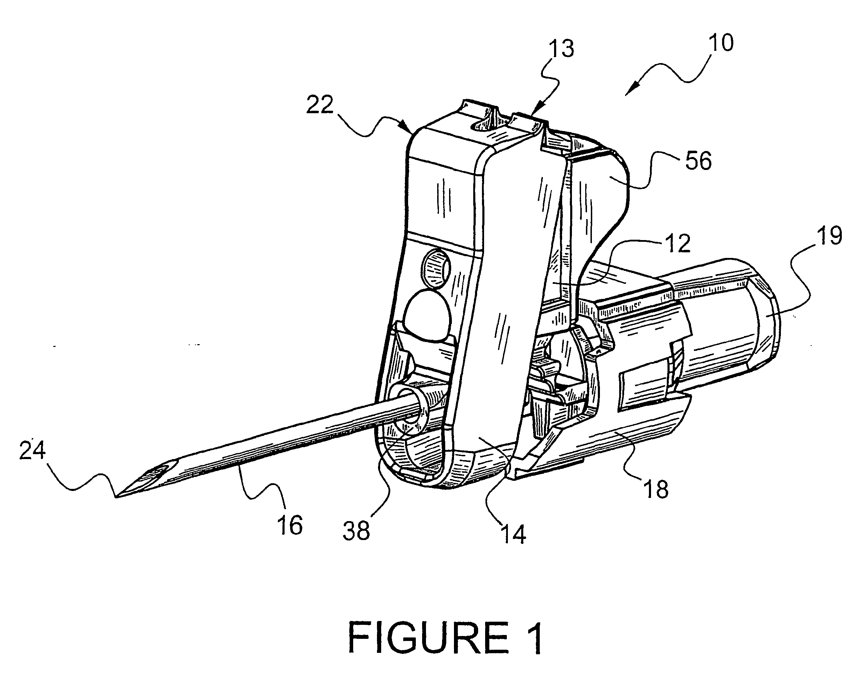

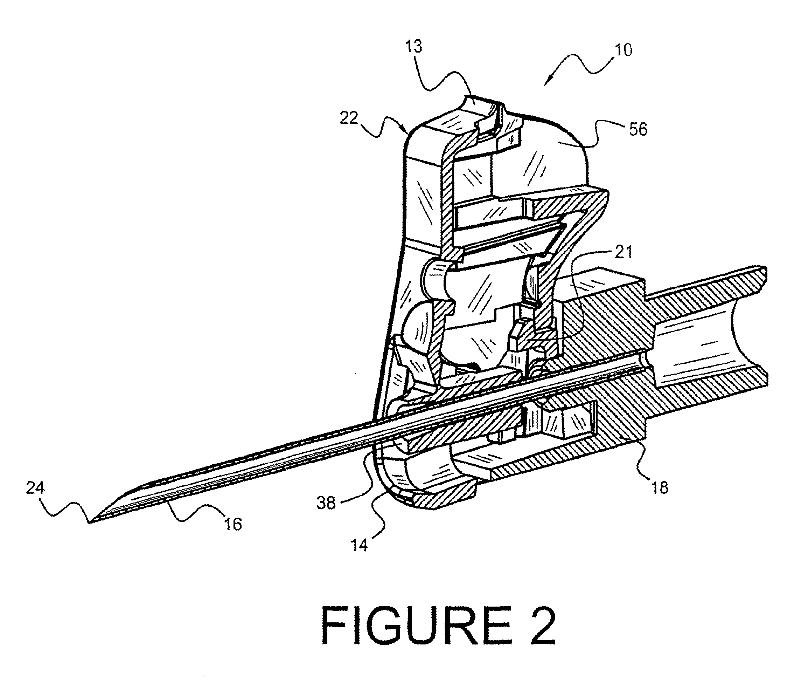

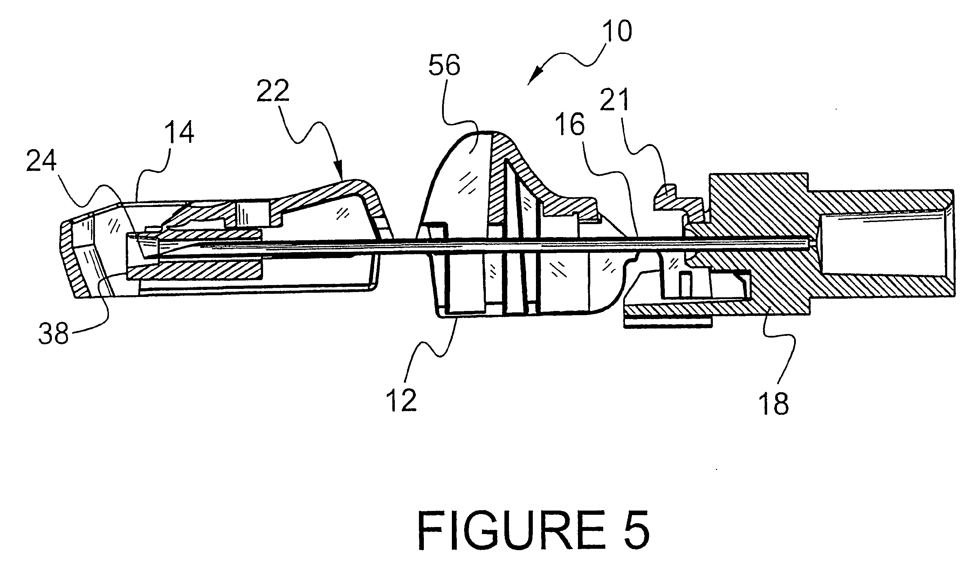

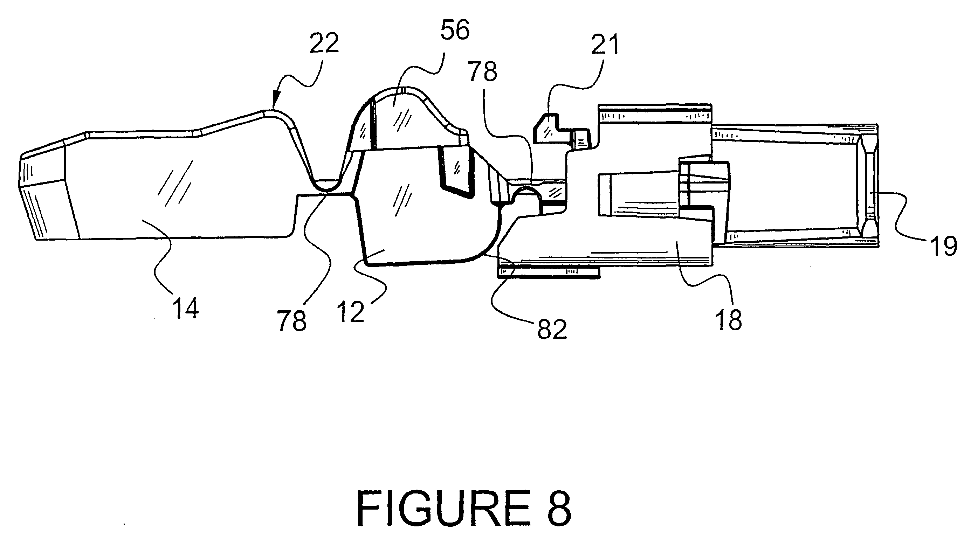

In this description, the term proximal is generally used to indicate relative nearness of a referenced item to a user of a device or a viewer of a perspective drawing of a FIGURE. The term distal is similarly used to indicate relative remoteness. Reference is now made to the embodiments illustrated in FIGURES 1- 21 wherein like numerals are used to designate like parts throughout. In cases where parts have similar, but not identical, form and function, numerals with primes may be used for ease in interpretative cross referencing.

Referring to FIGURES 1-3, an embodiment of a safety shield apparatus 10 is shown comprising a safety shield 22 of hingedly connected segments 12 and 14 for protecting a needle 16 after use in a medical procedure. The needle 16 has a proximal end and a distal end 24 with the proximal end of the needle 16 being bonded with a hub 18. It is envisioned that needle 16 may be affixed to hub 18 in various manners. Safety shield apparatus 10 has a luer fitting 19 for attachment to various needle devices such as a syringe. It is contemplated that safety shield apparatus 10 may be utilized with other medical needle applications including, but not limited to, phlebotomy devices, catheters, catheter introducers, guide wire introducers, spinal and epidural, biopsy, apheresis, dialysis, blood donor, Veress needles, Huber needles, etc., and therefore, may incorporate a hub configuration other than a luer fitting.

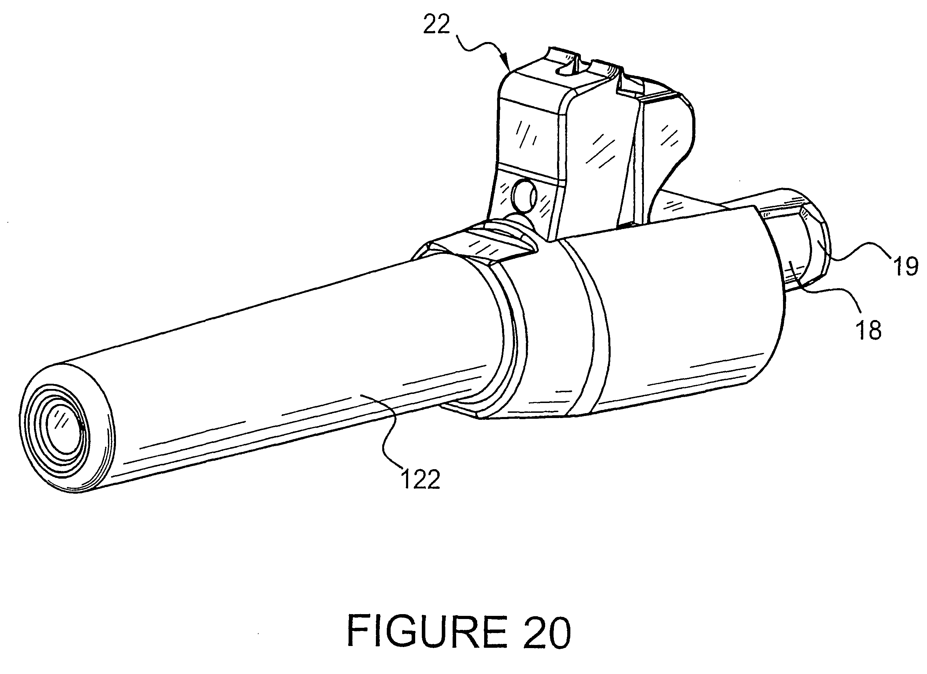

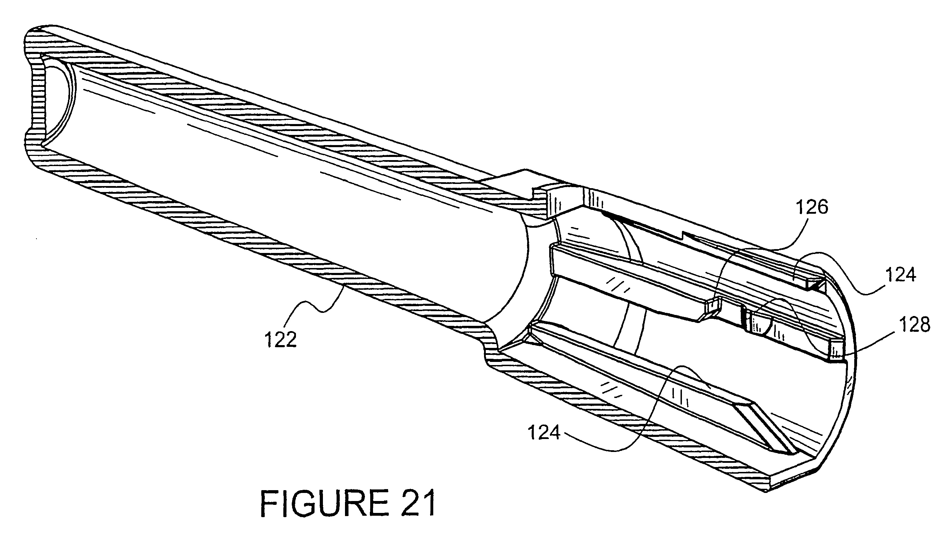

The molded components of safety shield apparatus 10 including the hub 18, shield 22 and a sheath 122, described below with regard to FIGURES 20 and 21, are designed to be molded without the need for side-pull cores.

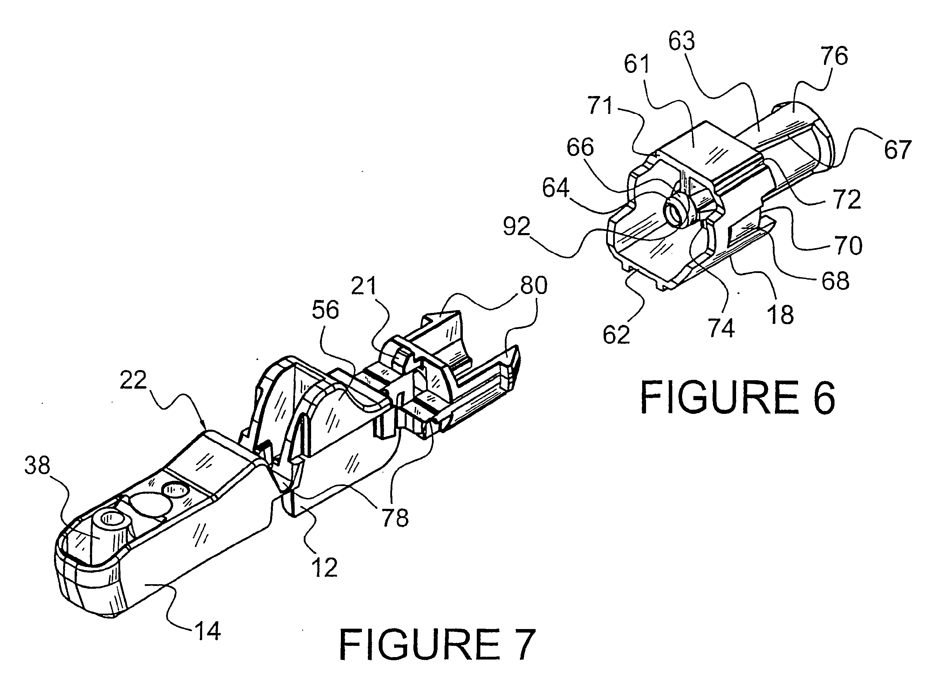

The distal end 24 of the needle 16 includes a bevel which may be aligned in a plane of symmetry with the shield 22 for indicating orientation of the bevel. The needle bevel may be oriented with respect to the retracted shield 22 to provide a consistent needle bevel configuration for a user. The shield 22 and hub 18 are connected through a bayonet-type snap fitment. A proximal end of shield 22 is received by a collar 61 of hub 18 wherein tabs, such as, for example, snaps 80 retain shield 22 to the hub 18 by interlocking with notches 70, as shown in FIGURES 6 and 7. Snaps 80 have a prong-like configurations. Snaps 80 may have other configurations, such as, for example, detents, clips, etc. It is contemplated that snaps 80 flexibly extend from shield 22 to engage an inner surface of collar 61 and resiliently project through notches 70 to interlock therewith. Collar 61 has a substantially cylindrical configuration. It is envisioned that collar 61 may have a variety of geometric configurations, such as, for example, rectangular, polygonal, etc. It is further envisioned that collar 61 may have various dimensions of length, diameter, width, etc.

Significant cost savings may result if a manufacturing mold is constructed from two simple plates which separate along a common axis and remain parallel to each other. In general, this requires that all part surfaces be formed by planes which form angles from ninety to one hundred eighty degrees with the mold parting surface; and if the part tapers, it should taper in such a manner as to get smaller in dimension proceeding along an axis into the mold cavity. This taper is referred to as "mold draft" and prevents the part geometry from being trapped in the mold geometry. This is commonly referred to as a "straight pull" mold.

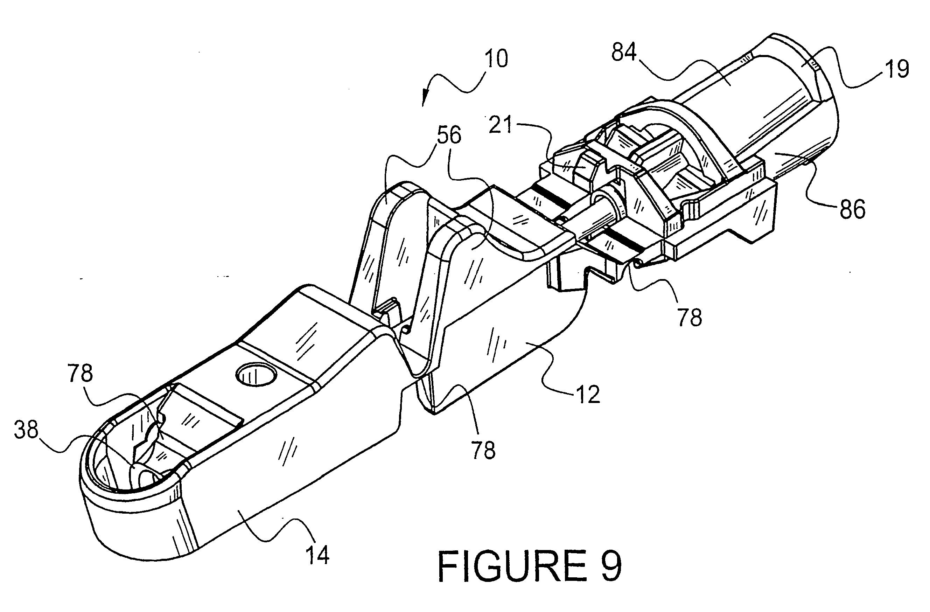

Notches 70 interlock with snaps 80 to form a snap fit component. Notches 70 are more difficult to mold in a straight pull fashion, since they may become trapped in the mold. Wedge features 76 and 86 provide for straight pull molding of this feature, while not adversely affecting the function of the luer fitting 19. Wedge features 76 and 86 (FIGURE 9) provide this function through surfaces 63, which are parallel to the axis of the mold separation, and surfaces 67 which taper to form a seal or shutoff with the mating half of the mold surfaces 67 may also be parallel. Tapering or drafting of surfaces 63 in an expanding fashion should be avoided, which would cause interference with the lock ring of a standard syringe. Wedge features 76 and 86 allow the mold to form the latch feature, e.g., notches 70 (FIGURES 6 and 7).

A retention catch 21, formed on the proximal end of shield 22, releasably latches with proximal segment 12 to hold the shield 22 in a fully retracted position for use. Proximal segment 12 has a surface which slides over retention catch 21 and is retained thereby in a latching or catch configuration. It is contemplated that retention catch 21 may engage various portions of proximal segment 12. It is further contemplated that shield 22 may be retained by multiple detents or retention catches of the proximal end of shield 22. Other latching configurations are also envisioned such as, for example, pins, clips, etc. Shield 22 may also be held in the retracted position via engagement with hub 18.

The shield 22 is manually extended and locked in a single-handed manner following use by either: 1) pushing the shield 22 with a finger, for example at raised surface 56 (FIGURE 9); or 2) surface activation by, for example, pushing the shield 22 against a surface such as a tabletop. Referring to FIGS. 1-3, surface activation is enabled due to the configuration of shield 22 such that proximal segment 12 and distal segment 14 form a general fulcrum point 13 engageable to extend shield 22 to the extended position. Fulcrum 13 includes a hinge portion projecting from shield 22 that engages the table, etc. It is contemplated that fulcrum 13 may include hinge portions, such as, for example, living hinges, pinned hinges, etc. This surface activation configuration advantageously facilitates one-handed operation and does not require the above-discussed finger actuation.

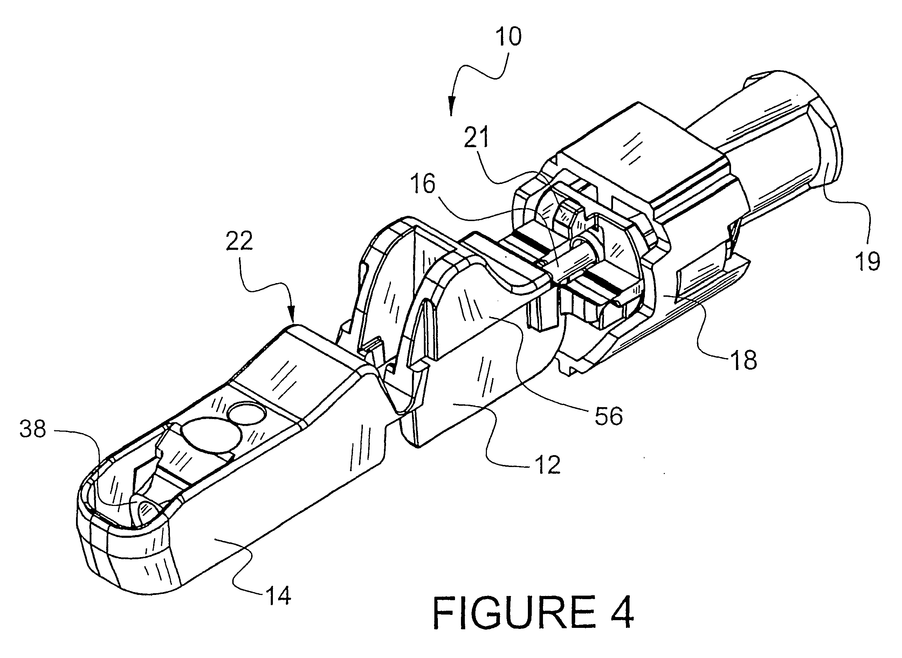

Referring to FIGURES 1, 3 and 4, shield 22 is extendable from a retracted position (FIGURE 1) to an extended position (FIGURE 4). The shield 22 irreversibly locks around the needle 16 upon full extension to protect the user from inadvertent exposure to the needle point 24.

Referring to FIGURES 6 and 7, a surface, such as, for example, an over-travel stop 62 on the hub 18 contacts surface 82 on the proximal segment 12. Stop 62 limits rotation of proximal segment 12 relative to hub 18 to advantageously preclude excess bending of the needle 16 during and after full extension of the shield 22 to the needle 16. Stop 62 extends from hub 18 forming a planar edge configured to engage shield 22, thereby limiting rotation of proximal segment 18 and consequently, needle 16. It is contemplated that stop 62 may have various configurations for engaging shield 22, such as, for example, staggered, stepped, interlocking, offset, etc. It is further contemplated that the over-travel stop may be formed with shield 22. It is envisioned that stop 62 limits undesirable rotation of shield 22, such as, for example, over-rotation, rotation that causes plastic deformation of needle 16, etc.

Collar 61 provides for convenient and safe grasping of the hub. This ergonomic feature of the present disclosure advantageously provides a surface that attracts users to grasp collar 61 for syringe removal, etc. It is envisioned that hub 18 may include other ergonomic features such as color coding. Surfaces 64, 66 and 89, 90 provide for guiding the hub 18 into the correct position with the shield 22. The shield 22 abuts against the hub 18 at surface 71 when in the retracted position.

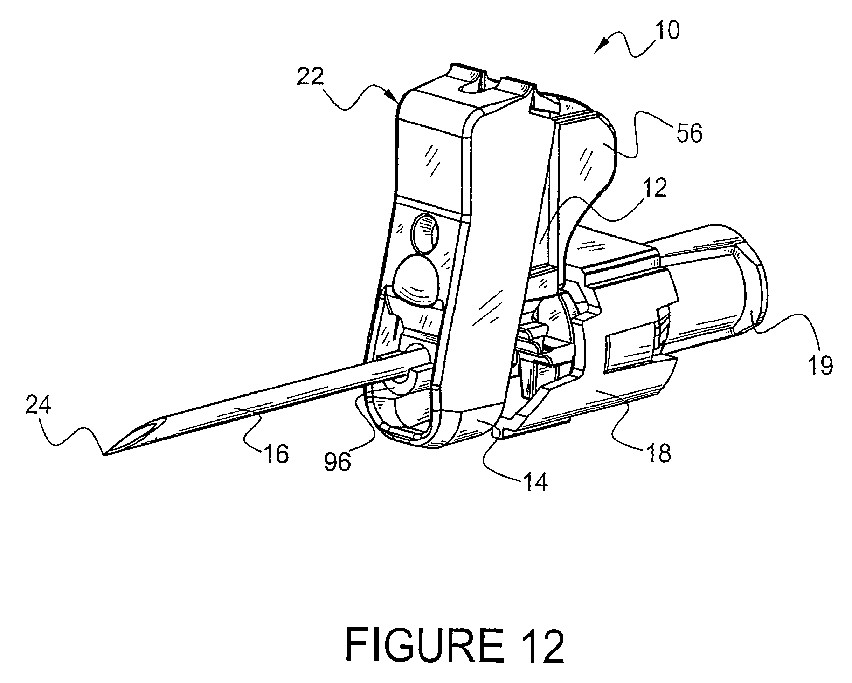

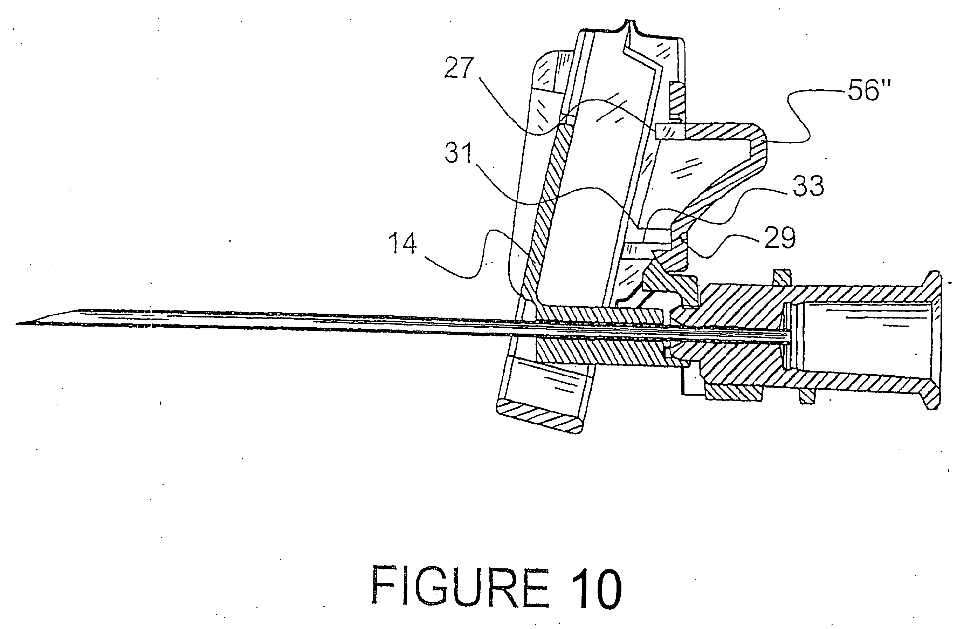

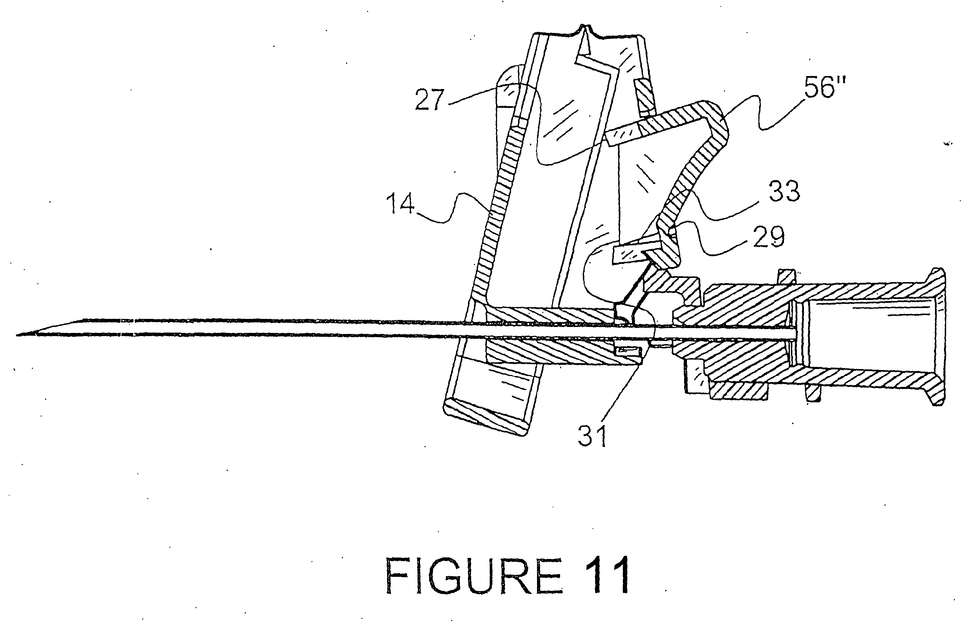

In an alternate embodiment, the raised surface 56 (FIGURE 12), as an aid in urging the shield 22 to the extended position may be further configured to form an articulating actuator 56" as shown in FIGURES 10 and 11. The articulating actuator 56" may pivot about a hinge 29 and may further be biased in such a way as to maintain the articulating actuator 56" in a relaxed position (FIGURE 10). During actuation, the actuation force on the articulating actuator 56" acts directly upon a segment other than the proximal segment 12 (the distal segment 14 in FIGURE 11) at contact surface 27, thereby, enhancing advancement of the shield 22. Stop surfaces 31 and 33 maybe provided between the articulating actuator 56" and its segment to prevent over-travel of the articulating actuator 56".

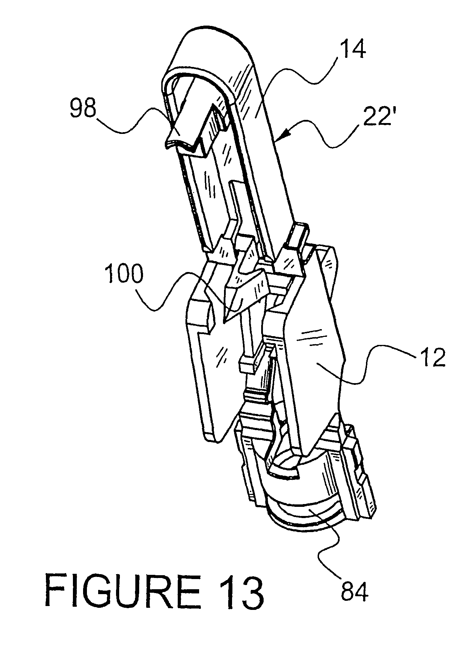

Referring back to FIGURES 1-5, a linear bearing 38 is hingedly disposed within the distal segment 14 and slides linearly along the needle 16 as the distal segment 14 translatably rotates along the needle 16 when the shield 22 is extended from the retracted position to the extended position. The linear bearing 38 shields the distal end 24 of the needle 16 when the shield 22 is in the extended position. Linear bearing 38 fully covers distal end 24 to provide an increased perception of security and minimization of fluid splatter when the shield is extended and locked. Referring to FIGURE 12, an alternate embodiment illustrates a duckbill-type linear bearing 96, which allows the distal end 24 of the needle 16 to be visible through the top of the shield 22. This configuration also beneficially provides point protection perception to a user. Referring to FIGURE 13, another alternate embodiment illustrates a flap 98, employable with linear bearing 38 or 96, for aligning the linear bearing 38 or 96, during assembly.

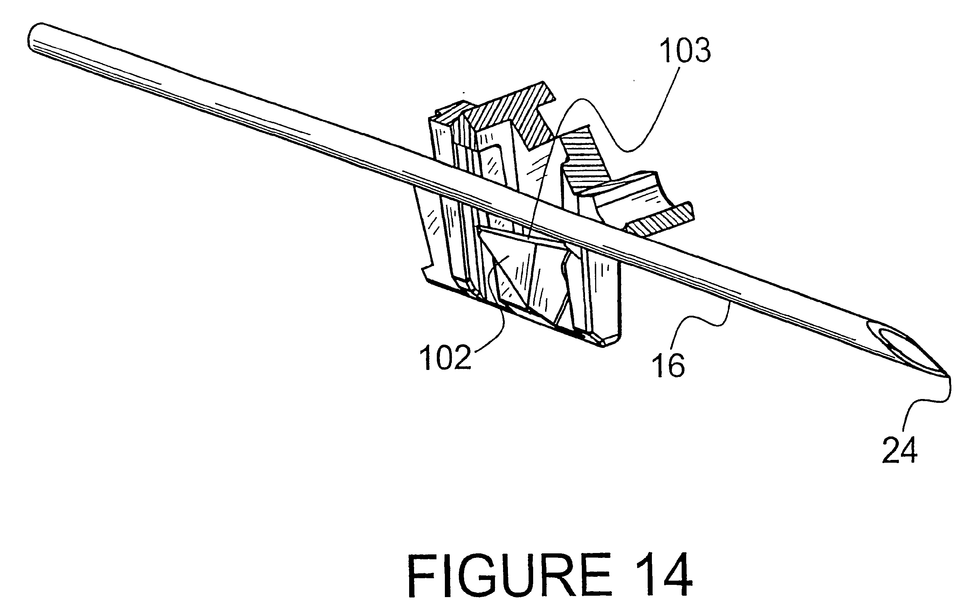

Referring to FIGURE 13, safety shield apparatus 10 has a barbed flap lock 100, which snaps around the needle 16 when the shield 22 is fully extended to lock shield 22 and retain needle 16 in a protective configuration providing security for a user from accidental needle stick. Referring to FIGURE 14, an alternate embodiment shows an angled flap lock 102 that is advantageous for a wide range of needle 16 sizes to capture small gage needles and larger gage needles. The angled flap lock 102 easily flexes to facilitate needle 16 capture. The bottom edge of the angled flap lock 102 is angled to drive the needle 16 in toward the root of the angled flap lock 102 when the needle 16 is pulled back against the angled flap lock 102. This results in a lock with a light locking force and considerable retention force. Further, flap lock 102 has a reduced mass, facilitating placement within smaller spaces. This configuration makes flap lock 102 difficult to defeat, precluding easy reset. The angled flap lock 102 is also advantageous in its ability to limit movement of the needle 16 relative to the shield 22 due to the interaction of the flat lower surface 103 of the angled flap lock 102 with the needle 16 in the latched condition.

The hinges connecting segments 12 and 14 may be flexible living hinges 78, pinned hinges, or equivalents thereof that provide for hinged connections of the segments 12 and 14. However, the number of hingedly connected segments depends upon the needle 16 length and device length required to extend the shield 22 beyond the distal end 24 of the needle 16. The present invention may be injection molded using polypropylene, other synthetic resinous materials, or equivalents thereof that provide for fabrication of living hinges 78.

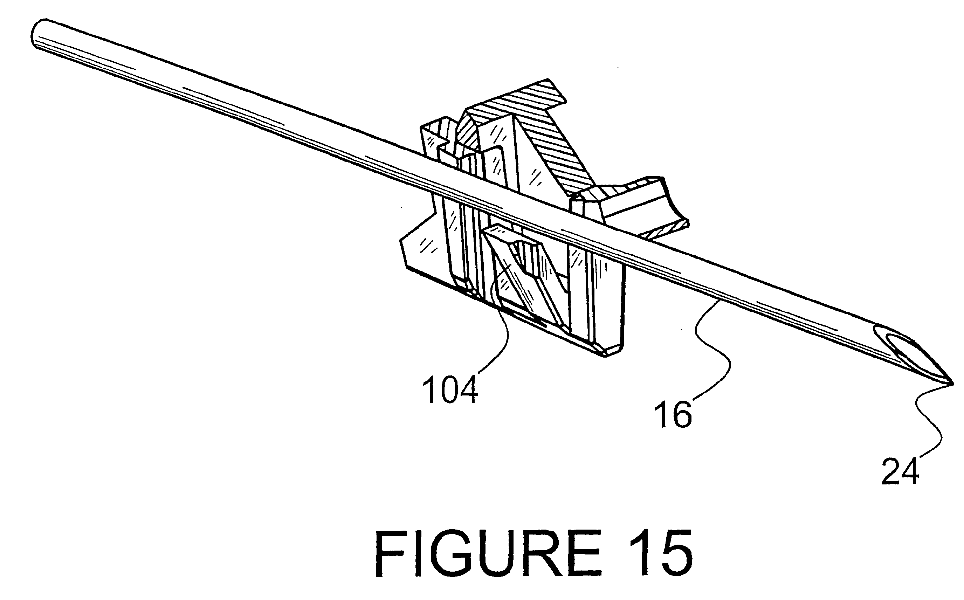

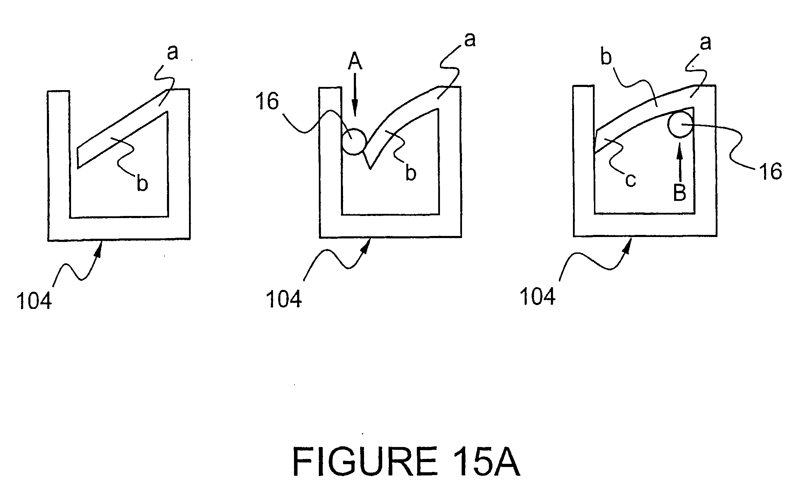

In an alternate embodiment, the needle lock includes a rectangular flap lock 104 as shown in FIGURES 15 and 15A. The rectangular flap lock 104 is configured to lock with low latching forces for small gage needles, while maintaining robust retention forces for larger gage needles. The rectangular lock comprises an angled rectangular flap lock 104 molded as part of one side of the shield 22. The rectangular flap lock 104 is designed to flex inward toward an attachment point a upon needle 16 engagement with lock arm *b*, as shown by arrow A. The needle 16 is forced back to the root (attachment point *a*) of the rectangular flap lock 104 when pulled back against lock arm *b* of the rectangular flap lock 104, as shown by arrow B. As the pullback force increases, the lock arm *b* of rectangular flap lock 104 may deflect until a free end *c* of the rectangular flap lock 104 contacts the wall opposite the root of the rectangular flap lock 104. This effectively supports the latch in two places: the root and the contact point of free end c with the opposite wall. The end result is a lock that has relatively low engagement forces and substantial retention forces. The thickness of lock 104 is reduced adjacent attachment point *a* relative to lock arm b. This configuration advantageously minimizes bending of needle 16, and facilitates placement within smaller spaces making flap lock 104 difficult to defeat, precluding easy reset.

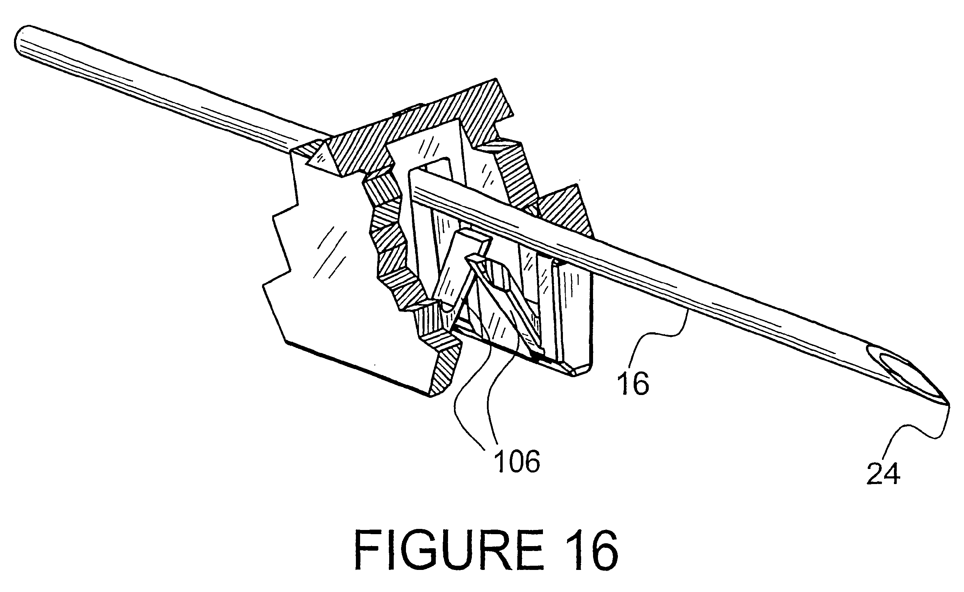

In another embodiment, the needle lock is a double flap lock 106 as shown in FIGURE 16. This lock configuration consists of two rectangular locks, each attached to opposite walls on the shield 22. This lock substantially increases the difficulty in manually defeating the lock, advantageously precluding reset because both locking flaps of the double flap lock 106 must be defeated simultaneously to reset the lock.

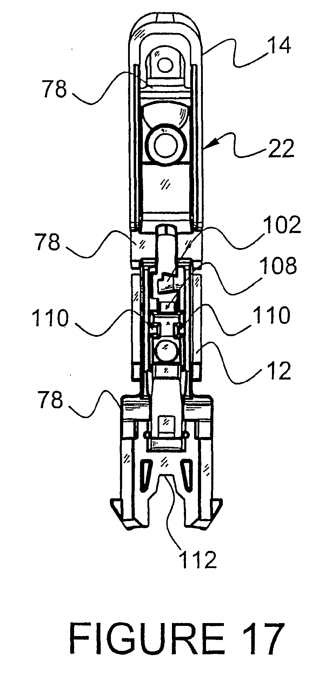

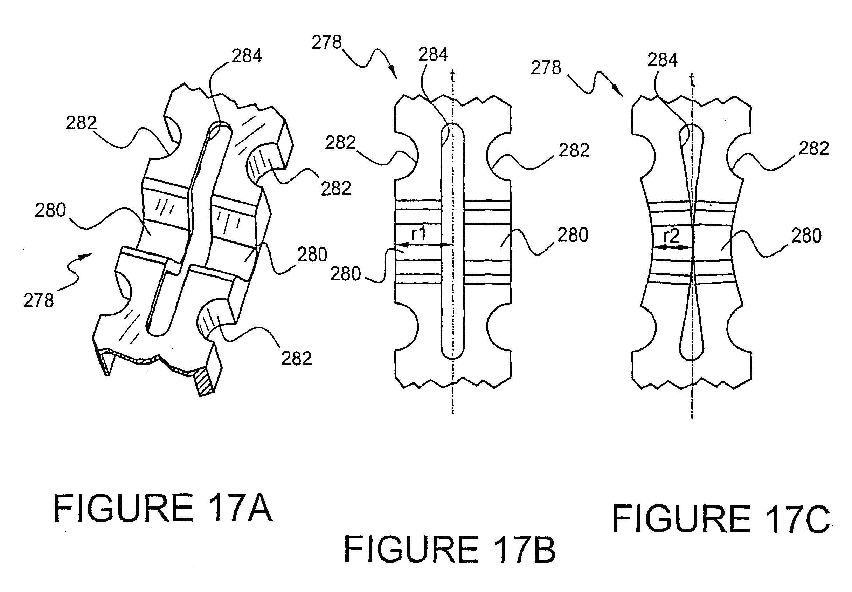

Additional features may be added to the locks of the present disclosure to increase the difficulty in manually defeating the needle locks and thereby preclude easy reset. For example, referring to FIGURE 17, one or more stiffening ribs 110 may be added to either side of the lock to minimize finger contact with the locking flap and to stiffen the shield 22 walls to make wall deflection more difficult. A stiffening arch 108 may be added to minimize shield 22 wall spreading and to make direct finger tip access to the lock more difficult. Moreover, for example, by rotating the angled flap lock 102 so as to place the free end of the angled flap lock 102 away from the living hinge 78 (at the end of the distal end of proximal segment 12), the potential for fingertip access to the angled flap lock 102 is minimized.

Referring to FIGURES 17A-17C, in an alternate embodiment, living hinges 278 may provide hinged connections for segments 12 and 14 of shield 22 and hub 18. The areas around living hinges 278 are relieved by relieved portions, discussed below, to enable living hinges 278 to flex inward toward needle 16 when shield 22 is rotated in torsion, such as, for example, by twisting. Living hinge 278, which can be disposed between the segments and/or the hub, has a pair of hinge straps 280 that extend between the members being hinged. It is contemplated that one or a plurality of hinge straps 280 may be employed.

The relieved portions include crumple zones, such as, for example, reliefportions 282 and 284. Relief portions 282 are formed on opposing sides of living hinge 278 in an outer surface thereof. It in envisioned that one or a plurality of relief portions 282 may be used. Relief portion 284 is formed within living hinge 278 as a cavity extending along the longitudinal length thereof. Relief portion 284 may be variously configured according to geometry, dimension, etc., in accordance with the principles of the present disclosure and suitability for a particular medical needle application. Relief portions 282 and 284 are configured to cause living hinges 278 to flex inward for accommodating greater amounts of stress due at least in part to torsion, prior to failure, such as, for example, plastic deformation, fracture, etc., as will be discussed.

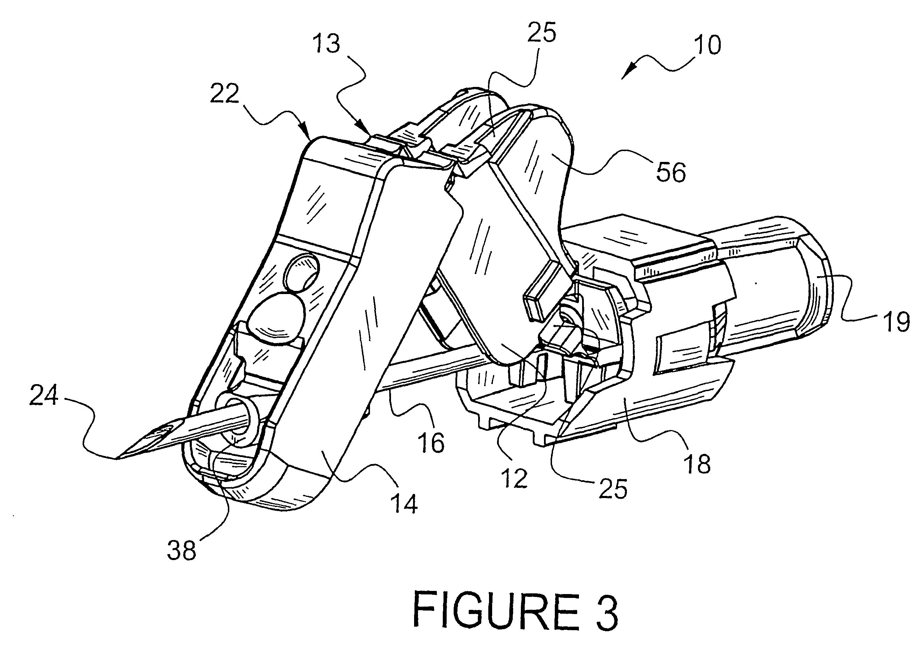

Referring to FIGURE 17B, the stress in living hinge 278 including hinge straps 280 at any given point is in direct proportion to a distance, such as, for example, a radius r1 of that point from an axis *t* of torsion. Consequently, the greatest amount of stress is at the outermost edges of hinge straps 280. Prior to application of torsion, e.g., twisting of living hinge 278, the stress on hinge straps 280 is approximately zero. As living hinge 278 is twisted, a stress is created in hinge straps 280. Referring to FIGURE 17C, reliefs 282 and 284 facilitate hinge strap's 280 move closer to axis *t* such that the distance from axis *t*, r2, is reduced, thereby reducing the stress at r2, in accordance with that described above. This configuration advantageously increases the amount of rotational deflection necessary to cause hinge failure in torsion due to twisting. Further, an indication of failure is provided to a user. Alternatively, to increase the amount of twisting necessary to cause the living hinges 78 of the actuated device to fail, support structures 25 (FIGURE 3) in the form of thin plates in a plane perpendicular to the plane of the hinges 78 may be added. These support structures 25 may take the form of living hinges, but are not limited to such forms.

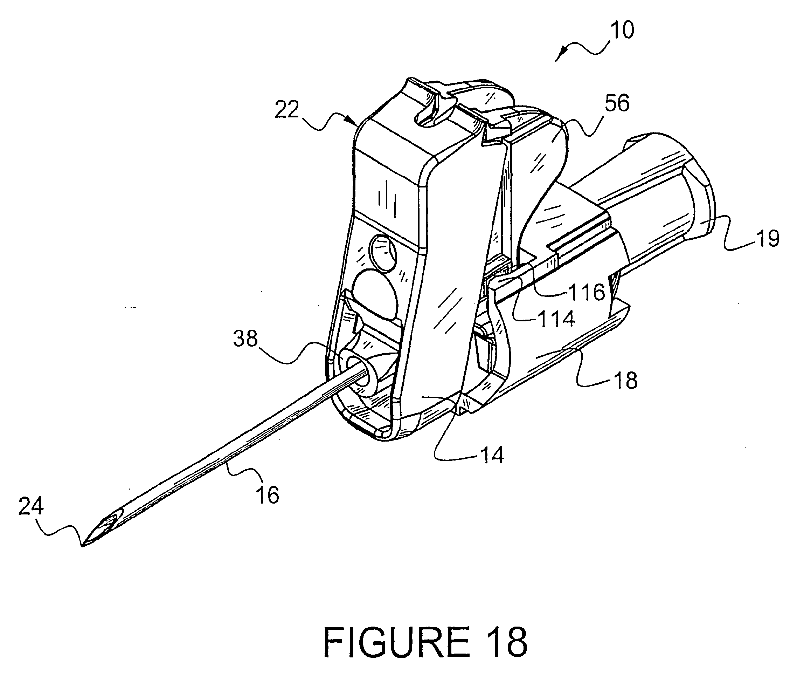

As shown in FIGURE 18, a collar clip detent 114 may be added to the hub 18 that engage a recessed area 116 for retaining the shield 22 in the retracted state. An alternate embodiment may be a detail on the shield 22 that slides under the hub collar (not shown).

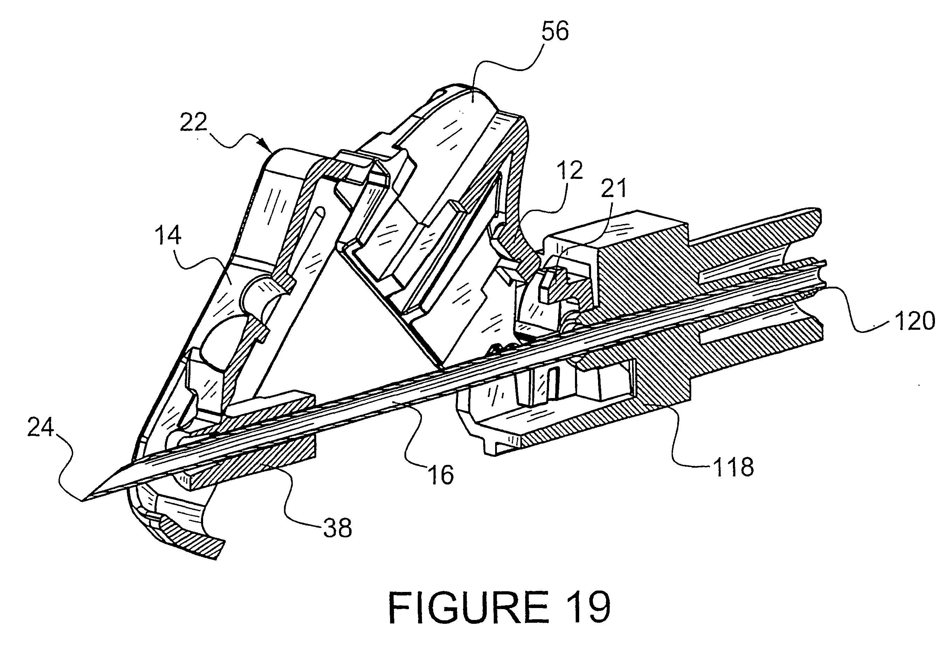

In an alternate embodiment, FIGURE 19 illustrates a hub 118 having an extended needle enclosure 120. The hub 118 is advantageous for applications where a syringe is used to withdraw medications from a drug vial in that enclosure 120 reduces the dead volume in the hub 118.

Referring to FIGURES 20 and 21, a sheath 122 is slidably connected to the hub 18 to cover the needle 16 prior to use. Rails 124 guide the hub 18 into place as the safety shield apparatus 10 is slid into the sheath 122. A notched section 72 (FIGURE 6) of the hub 18 interfaces with rails 124. It is envisioned that one or multiple rails may be employed. A hub stop 126 and detent bump 128 engage the hub 18 for retaining the sheath 122 in place prior to use. A rib 74 (FIGURE 6) disposed on the hub 18 slides into the notch 112 (FIGURE 17) in the shield 22 to provide for the proper alignment of the shield 22 as it is slidably connected to the hub 18.

In another embodiment the hub may be configured to include a luer fitting for attachment to various needle devices such as a syringe or IV set.

The invention of the present disclosure may be embodied in other specific forms. The present embodiments are therefore to be considered in all respects as illustrative and not restrictive.

## Claims

1. A medical needle shield apparatus comprising:
a monolithic needle hub (18) configured to receive a needle and including a collar (61) defining an interior cavity; and
a shield (22) having a distal end and a proximal end receivable by the interior cavity of the collar (61) in an interlocking engagement, the shield (22) including a plurality of hingedly connected segments (12, 14) and being extensible between a retracted position and an extended position, **characterized in that** the interior cavity defines notches (70) that receive tabs (80) formed with the proximal end of the shield (22) to lock the needle hub (18) with the shield (22).

2. The medical needle shield apparatus as recited in claim 1, further comprising at least two surfaces which engage to limit rotation of the shield (22) relative to the needle hub (18).

3. The medical needle shield apparatus as recited in claim 1, wherein the distal end of the shield (22) encloses at least a portion of the distal end of the needle when the shield is in the extended position.

4. The medical needle shield apparatus as recited in claim 1, wherein the tabs (80) are biased for receipt within the notches.

5. The medical needle shield apparatus as recited in claim 3, wherein the shield (22) is locked in the extended position.

6. The medical needle shield apparatus as recited in claim 3, wherein the shield (22) is irreversibly locked in the extended position.

7. The medical needle shield apparatus as recited in claim 3, wherein the shield (22) is locked in the extended position via engagement with the needle.

8. The medical needle shield apparatus as recited in claim 3, wherein the shield (22) includes a lock that engages the needle to lock the shield in the extended position.

9. The medical needle shield apparatus as recited in claim 8, wherein the lock includes a portion configured to flexibly engage the needle and biased to lockably retain the needle.

10. The medical needle shield apparatus according to claim 3, wherein at least one segment includes at least one linear bearing for facilitating extension of the segments when extending the shield over the needle.

11. The medical needle shield apparatus as recited in claim 10, wherein the linear bearing (38) is hingedly connected to the shield (22) proximal to the distal end of the shield.

12. The medical needle shield apparatus as recited in claim 10, wherein the linear bearing (38) is configured such that at least a portion of the needle is enclosed by the shield (22) during extension of the shield.

13. The medical needle shield apparatus as recited in claim 3, wherein the needle hub (18) has a luer fitting configured to attach to a syringe.

14. The medical needle shield apparatus as recited in claim 3, wherein the shield (22) includes a proximal segment engaging a retention catch formed with the proximal end of the shield (22) to releasably dispose the shield in the retracted position.

15. The medical needle shield apparatus as recited in claim 3, further comprising a sheath engageable with the needle hub (18).

16. The medical needle shield apparatus as recited in claim 15, wherein the sheath has guide rails configured to facilitate engagement of the sheath and the needle hub (18).

17. The medical needle shield apparatus as recited in claim 3, wherein the shield (22) has an articulating actuator configured to urge the shield towards the extended position.

18. The medical needle shield apparatus as recited in claim 3, further comprising a tape down member attached to the shield (22) and configured to facilitate extension of the shield (22).

19. The medical needle shield apparatus as recited in claim 3, wherein the needle hub (18) includes guide surfaces to facilitate engagement of the shield (22) and the needle hub (18).

20. The medical needle shield apparatus as recited in claim 3, wherein the needle hub (18) includes at least one catch and the shield (22) includes at least one corresponding protrusion which engage to lock the shield (22) in the extended position.

21. The medical needle shield apparatus as recited in claim 3, wherein the segments are connected via living hinges.

22. The medical needle shield apparatus as recited in claim 21, wherein the segments include relief portions formed adjacent the living hinges.

23. The medical needle shield apparatus as recited in claim 22, wherein the relief portions are configured to flex inwardly toward the needle.

24. The medical needle shield apparatus according to claim 3, wherein at least one segment includes at least one rib for positioning the needle.

25. The medical needle shield apparatus as recited in claim 24, wherein the at least one rib has a transverse orientation.

26. The medical needle shield apparatus as recited in claim 3, configured for use with a port access needle.

27. The medical needle shield apparatus as recited in claim 26, wherein a pair of wings are attached to the proximal end of the shield (22).

28. The medical needle shield apparatus as recited in claim 26, wherein the shield (22) includes a needle latch that engages the needle in the extended position.

29. The medical needle shield apparatus according to claim 3, wherein the hingedly connected segments are connected by pinned hinges.

30. The medical needle shield apparatus according to claim 8, wherein the lock includes at least one catch for engagement with a corresponding protrusion disposed on the shield (22) in the extended position.

31. The medical needle shield apparatus according to claim 8, wherein the lock includes at least one catch for engagement with a corresponding protrusion disposed on the hub in the extended position.

32. The medical needle shield apparatus according to claim 8, wherein the lock includes at least one protrusion for engagement with a corresponding catch disposed on the shield (22) in the extended position.

33. The medical needle shield apparatus according to claim 8, wherein the lock includes at least one protrusion for engagement with a corresponding catch disposed on the hub in the extended position.

34. The medical needle shield apparatus according to claim 3, further comprising a latch which secures a distal segment of the shield (22) in the extended position, the distal segment having an underside including a surface extending over at least a portion of the distal segment for retaining the distal end of the needle.

35. The medical needle shield apparatus according to claim 3, further comprising a retainer for holding the segments in a retracted position.

36. The medical needle shield apparatus according to claim 35, wherein the retainer includes a retainer arm disposed on the needle hub (18) and extending to a corresponding catch disposed on the shield (22) in the retracted position.

37. The medical needle shield apparatus according to claim 35, wherein the retainer includes at least one retention catch disposed on the segments.

38. The medical needle shield apparatus according to claim 3, wherein the shield (22) further includes a raised surface for urging the shield to the extended position.

39. The medical needle shield apparatus according to claim 3, wherein the needle comprises an open-ended needle.

40. The medical needle shield apparatus according to claim 3, wherein the needle comprises a double wall needle.

41. The medical needle shield apparatus according to claim 3, wherein the distal end of the needle includes a bevel aligned in a plane of symmetry with the shield (22) for indicating orientation of the bevel.

## Patentansprüche

1. Schutzvorrichtung für medizinische Nadeln, die Folgendes umfasst:
einen einstückigen Nadelansatz (18), der zur Aufnahme einer Nadel konfiguriert ist und einen einen inneren Hohlraum definierenden Bund (61) enthält; und
ein Schutzschild (22), das ein distales Ende und ein proximales Ende, das in einem Verriegelungseingriff durch den inneren Hohlraum des Bunds (61) aufgenommen werden kann, aufweist, wobei das Schutzschild (22) mehrere angelenkte Segmente (12, 14) enthält und zwischen einer zurückgezogenen Position und einer ausgezogenen Position ausziehbar ist, **dadurch gekennzeichnet, dass** der innere Hohlraum Kerben (70) definiert, die mit dem proximalen Ende des Schutzschildes (22) ausgebildete Nasen (80) aufnehmen, um den Nadelansatz (18) mit dem Schutzschild (22) zu verriegeln.

2. Schutzvorrichtung für medizinische Nadeln nach Anspruch 1, die weiterhin mindestens zwei Flächen umfasst, die in Eingriff gelangen, um eine Drehung des Schutzschildes (22) bezüglich des Nadelansatzes (18) zu begrenzen.

3. Schutzvorrichtung für medizinische Nadeln nach Anspruch 1, wobei das distale Ende des Schutzschildes (22) mindestens einen Teil des distalen Endes der Nadel einschließt, wenn sich das Schutzschild in der ausgezogenen Position befindet.

4. Schutzvorrichtung für medizinische Nadeln nach Anspruch 1, wobei die Nasen (80) zur Aufnahme in den Kerben vorgespannt sind.

5. Schutzvorrichtung für medizinische Nadeln nach Anspruch 3, wobei das Schutzschild (22) in der ausgezogenen Position verriegelt ist.

6. Schutzvorrichtung für medizinische Nadeln nach Anspruch 3, wobei das Schutzschild (22) irreversibel in der ausgezogenen Position verriegelt ist.

7. Schutzvorrichtung für medizinische Nadeln nach Anspruch 3, wobei das Schutzschild (22) über einen Eingriff mit der Nadel in der ausgezogenen Position verriegelt ist.

8. Schutzvorrichtung für medizinische Nadeln nach Anspruch 3, wobei das Schutzschild (22) eine Verriegelung enthält, die die Nadel in Eingriff nimmt, um das Schutzschild in der ausgezogenen Position zu verriegeln.

9. Schutzvorrichtung für medizinische Nadeln nach Anspruch 8, wobei die Verriegelung einen Teil enthält, der dazu konfiguriert ist, die Nadel flexibel in Eingriff zu nehmen, und zum verriegelbaren Festhalten der Nadel vorgespannt ist.

10. Schutzvorrichtung für medizinische Nadeln nach Anspruch 3, wobei mindestens ein Segment mindestens ein Linearlager enthält, um das Ausziehen der Segmente beim Ausziehen des Schutzschildes über die Nadel zu erleichtern.

11. Schutzvorrichtung für medizinische Nadeln nach Anspruch 10, wobei das Linearlager (38) nahe dem distalen Ende des Schutzschildes am Schutzschild (22) angelenkt ist.

12. Schutzvorrichtung für medizinische Nadeln nach Anspruch 10, wobei das Linearlager (38) so konfiguriert ist, dass mindestens ein Teil der Nadel beim Ausziehen des Schutzschildes vom Schutzschild (22) eingeschlossen ist.

13. Schutzvorrichtung für medizinische Nadeln nach Anspruch 3, wobei der Nadelansatz (18) einen Luer-Anschluss aufweist, der zur Befestigung an einer Spritze konfiguriert ist.

14. Schutzvorrichtung für medizinische Nadeln nach Anspruch 3, wobei das Schutzschild (22) ein proximales Segment enthält, das eine Haltearretierung in Eingriff nimmt, die mit dem proximalen Ende des Schutzschildes (22) ausgebildet ist, um das Schutzschild in der zurückgezogenen Position freigebbar anzuordnen.

15. Schutzvorrichtung für medizinische Nadeln nach Anspruch 3, die weiterhin eine mit dem Nadelansatz (18) in Eingriff bringbare Hülse umfasst.

16. Schutzvorrichtung für medizinische Nadeln nach Anspruch 15, wobei die Hülse Führungsschienen aufweist, die dazu konfiguriert sind, den Eingriff der Hülse und des Nadelansatzes (18) zu erleichtern.

17. Schutzvorrichtung für medizinische Nadeln nach Anspruch 3, wobei das Schutzschild (22) ein Gelenk-Stellglied aufweist, das dazu konfiguriert ist, das Schutzschild in die ausgezogene Position zu drücken.

18. Schutzvorrichtung für medizinische Nadeln nach Anspruch 3, die weiterhin ein Befestigungsglied umfasst, das am Schutzschild (22) angebracht und dazu konfiguriert ist, das Ausziehen des Schutzschildes (22) zu erleichtern.

19. Schutzvorrichtung für medizinische Nadeln nach Anspruch 3, wobei der Nadelansatz (18) Führungsflächen enthält, um den Eingriff des Schutzschildes (22) und des Nadelansatzes (18) zu erleichtern.

20. Schutzvorrichtung für medizinische Nadeln nach Anspruch 3, wobei der Nadelansatz (18) mindestens eine Arretierung enthält und das Schutzschild (22) mindestens einen entsprechenden Vorsprung enthält, die das Schutzschild (22) in der ausgezogenen Position in Eingriff nehmen.

21. Schutzvorrichtung für medizinische Nadeln nach Anpruch 3, wobei die Segmente über Filmscharniere verbunden sind.

22. Schutzvorrichtung für medizinische Nadeln nach Anspruch 21, wobei die Segmente neben den Filmscharnieren ausgebildete Aussparungsteile enthalten.

23. Schutzvorrichtung für medizinische Nadeln nach Anspruch 22, wobei die Aussparungsteile dazu konfiguriert sind, sich nach innen zur Nadel zu biegen.

24. Schutzvorrichtung für medizinische Nadeln nach Anspruch 3, wobei mindestens ein Segment mindestens eine Rippe zur Positionierung der Nadel enthält.

25. Schutzvorrichtung für medizinische Nadeln nach Anspruch 24, wobei die mindestens eine Rippe eine Querausrichtung aufweist.

26. Schutzvorrichtung für medizinische Nadeln nach Anspruch 3, die zur Verwendung mit einer Port-Access-Nadel konfiguriert ist.

27. Schutzvorrichtung für medizinische Nadeln nach Anspruch 26, wobei ein Paar Flügel an dem proximalen Ende des Schutzschildes (22) befestigt ist.

28. Schutzvorrichtung für medizinische Nadeln nach Anspruch 26, wobei das Schutzschild (22) ein Nadelrastverriegelungsglied enthält, das die Nadel in der ausgezogenen Position in Eingriff nimmt.

29. Schutzvorrichtung für medizinische Nadeln nach Anspruch 3, wobei die angelenkten Segmente durch Bolzengelenke verbunden sind.

30. Schutzvorrichtung für medizinische Nadeln nach Anspruch 8, wobei die Verriegelung mindestens eine Arretierung zum Eingriff mit einem am Schutzschild (22) angeordneten entsprechenden Vorsprung in der ausgezogenen Position enthält.

31. Schutzvorrichtung für medizinische Nadeln nach Anspruch 8, wobei die Verriegelung mindestens eine Arretierung zum Eingriff mit einem am Ansatz angeordneten entsprechenden Vorsprung in der ausgezogenen Position enthält.

32. Schutzvorrichtung für medizinische Nadeln nach Anspruch 8, wobei die Verriegelung mindestens einen Vorsprung zum Eingriff mit einer am Schutzschild (22) angeordneten entsprechenden Arretierung in der ausgezogenen Position enthält.

33. Schutzvorrichtung für medizinische Nadeln nach Anspruch 8, wobei die Verriegelung mindestens einen Vorsprung zum Eingriff mit einer am Ansatz angeordneten entsprechenden Arretierung in der ausgezogenen Position enthält.

34. Schutzvorrichtung für medizinische Nadeln nach Anspruch 3, die weiterhin ein Rastverriegelungsglied umfasst, das ein distales Segment des Schutzschildes (22) in der ausgezogenen Position befestigt, wobei das distale Segment eine Unterseite aufweist, die eine Fläche enthält, welche sich über mindestens einen Teil des distalen Segments zum Festhalten des distalen Endes der Nadel erstreckt.

35. Schutzvorrichtung für medizinische Nadeln nach Anspruch 3, die weiterhin einen Halter zum Halten der Segmente in einer zurückgezogenen Position umfasst.

36. Schutzvorrichtung für medizinische Nadeln nach Anspruch 35, wobei der Halter einen Haltearm enthält, der am Nadelansatz (18) angeordnet ist und sich in der zurückgezogenen Position zu einer am Schutzschild (22) angeordneten entsprechenden Arretierung erstreckt.

37. Schutzvorrichtung für medizinische Nadeln nach Anspruch 35, wobei der Halter mindestens eine an den Segmenten angeordnete Haltearretierung enthält.

38. Schutzvorrichtung für medizinische Nadeln nach Anspruch 3, wobei das Schutzschild (22) weiterhin eine erhabene Fläche zum Drücken des Schutzschildes in die ausgezogene Position enthält.

39. Schutzvorrichtung für medizinische Nadeln nach Anspruch 3, wobei die Nadel eine Nadel mit offenem Ende umfasst.

40. Schutzvorrichtung für medizinische Nadeln nach Anspruch 3, wobei die Nadel eine doppelwandige Nadel umfasst.

41. Schutzvorrichtung für medizinische Nadeln nach Anspruch 3, wobei das distale Ende der Nadel eine Abschrägung enthält, die in einer Symmetrieebene auf das Schutzschild (22) ausgerichtet ist, um die Ausrichtung der Abschrägung anzuzeigen.

## Revendications

1. Appareil protecteur d'aiguille médicale comportant :
une embase (18) d'aiguille monolithique configurée pour recevoir une aiguille et comprenant un collier (61) définissant une cavité intérieure ; et
un protecteur (22) possédant une extrémité distale et une extrémité proximale recevables par la cavité intérieure du collier (61) dans une mise en prise d'interverrouillage, le protecteur (22) comprenant une pluralité de segments (12, 14) reliés par des charnières et pouvant s'étendre d'une position rétractée à une position déployée, **caractérisé en ce que** la cavité intérieure définit des encoches (70) qui reçoivent des languettes (80) formées avec l'extrémité proximale du protecteur (22) pour verrouiller l'embase (18) d'aiguille avec le protecteur (22).

2. Appareil protecteur d'aiguille médicale selon la revendication 1, comportant en outre au moins deux surfaces qui se mettent en prise pour limiter la rotation du protecteur (22) par rapport à l'embase (18) d'aiguille.

3. Appareil protecteur d'aiguille médicale selon la revendication 1, dans lequel l'extrémité distale du protecteur (22) enferme au moins une portion de l'extrémité distale de l'aiguille quand le protecteur est dans la position déployée.

4. Appareil protecteur d'aiguille médicale selon la revendication 1, dans lequel les languettes (80) sont contraintes à être reçues à l'intérieur des encoches.

5. Appareil protecteur d'aiguille médicale selon la revendication 3, dans lequel le protecteur (22) est verrouillé dans la position déployée.

6. Appareil protecteur d'aiguille médicale selon la revendication 3, dans lequel le protecteur (22) est verrouillé d'une manière irréversible dans la position déployée.

7. Appareil protecteur d'aiguille médicale selon la revendication 3, dans lequel le protecteur (22) est verrouillé dans la position déployée par sa mise en prise avec l'aiguille.

8. Appareil protecteur d'aiguille médicale selon la revendication 3, dans lequel le protecteur (22) comprend un verrou qui se met en prise avec l'aiguille pour verrouiller le protecteur dans la position déployée.

9. Appareil protecteur d'aiguille médicale selon la revendication 8, dans lequel le verrou comprend une portion configurée pour se mettre en prise de manière flexible avec l'aiguille et qui est contrainte à retenir l'aiguille d'une manière verrouillée.

10. Appareil protecteur d'aiguille médicale selon la revendication 3, dans lequel au moins un segment comprend au moins un palier linéaire pour faciliter le déploiement des segments lors du déploiement du protecteur sur l'aiguille.

11. Appareil protecteur d'aiguille médicale selon la revendication 10, dans lequel le palier (38) linéaire est relié au protecteur (22) par des charnières à proximité de l'extrémité distale du protecteur.

12. Appareil protecteur d'aiguille médicale selon la revendication 10, dans lequel le palier (38) linéaire est configuré de telle sorte qu'au moins une portion de d'aiguille est enfermée par le protecteur (22) au cours du déploiement du protecteur.

13. Appareil protecteur d'aiguille médicale selon la revendication 3, dans lequel l'embase (18) d'aiguille est munie d'un raccord Luer configuré pour y fixer une seringue.

14. Appareil protecteur d'aiguille médicale selon la revendication 3, dans lequel le protecteur (22) comprend un segment proximal se mettant en prise avec un cran de retenue formé avec l'extrémité proximale du protecteur (22) pour disposer d'une manière libérable le protecteur dans la position rétractée.

15. Appareil protecteur d'aiguille médicale selon la revendication 3, comportant en outre une gaine pouvant se mettre en prise avec l'embase (18) d'aiguille.

16. Appareil protecteur d'aiguille médicale selon la revendication 15, dans lequel la gaine est munie de rails de guidage configurés pour faciliter la mise en prise de la gaine avec l'embase (18) d'aiguille.

17. Appareil protecteur d'aiguille médicale selon la revendication 3, dans lequel le protecteur (22) possède un actionneur articulé configuré pour pousser le protecteur vers la position déployée.

18. Appareil protecteur d'aiguille médicale selon la revendication 3, comportant en outre un organe replié fixé sur le protecteur (22) et configuré pour faciliter le déploiement du protecteur (22).

19. Appareil protecteur d'aiguille médicale selon la revendication 3, dans lequel l'embase (18) d'aiguille comprend des surfaces de guidage pour faciliter la mise en prise du protecteur (22) avec l'embase (18) d'aiguille.

20. Appareil protecteur d'aiguille médicale selon la revendication 3, dans lequel l'embase (18) d'aiguille comprend au moins un cran et le protecteur (22) comprend au moins une saillie correspondante qui se met en prise pour verrouiller le protecteur (22) dans la position déployée.

21. Appareil protecteur d'aiguille médicale selon la revendication 3, dans lequel les segments sont reliés par des charnières mobiles.

22. Appareil protecteur d'aiguille médicale selon la revendication 21, dans lequel les segments comprennent des portions en relief formées à côté des charnières mobiles.

23. Appareil protecteur d'aiguille médicale selon la revendication 22, dans lequel les portions en relief sont configurées pour fléchir vers l'intérieur dans la direction de l'aiguille.

24. Appareil protecteur d'aiguille médicale selon la revendication 3, dans lequel au moins un segment comprend au moins une nervure pour positionner l'aiguille.

25. Appareil protecteur d'aiguille médicale selon la revendication 24, dans lequel ladite au moins une nervure a une orientation transversale.

26. Appareil protecteur d'aiguille médicale selon la revendication 3, configuré pour être utilisé avec une aiguille pour accès à chambre.

27. Appareil protecteur d'aiguille médicale selon la revendication 26, dans lequel une paire d'ailettes est fixée sur l'extrémité proximale du protecteur (22).

28. Appareil protecteur d'aiguille médicale selon la revendication 26, dans lequel le protecteur (22) comprend un cliquet d'aiguille qui met en prise l'aiguille dans la position déployée.

29. Appareil protecteur d'aiguille médicale selon la revendication 3, dans lequel les segments reliés par charnières sont reliés par des charnières à broche.

30. Appareil protecteur d'aiguille médicale selon la revendication 8, dans lequel le verrou comprend au moins un cran pour se mettre en prise avec une saillie correspondante disposée sur le protecteur (22) dans la position déployée.

31. Appareil protecteur d'aiguille médicale selon la revendication 8, dans lequel le verrou comprend au moins un cran pour se mettre en prise avec une saillie correspondante disposée sur l'embase dans la position déployée.

32. Appareil protecteur d'aiguille médicale selon la revendication 8, dans lequel le verrou comprend au moins une saillie pour se mettre en prise avec un cran correspondant disposé sur le protecteur (22) dans la position déployée.

33. Appareil protecteur d'aiguille médicale selon la revendication 8, dans lequel le verrou comprend au moins une saillie pour se mettre en prise avec un cran correspondant disposé sur l'embase dans la position déployée.

34. Appareil protecteur d'aiguille médicale selon la revendication 3, comportant en outre un cliquet qui immobilise de manière sécurisée un segment distal du protecteur (22) dans la position déployée, le segment distal ayant une face inférieure comprenant une surface s'étendant sur au moins une portion du segment distal pour retenir l'extrémité distale de l'aiguille.

35. Appareil protecteur d'aiguille médicale selon la revendication 3, comportant en outre un dispositif de retenue pour maintenir les segments dans une position rétractée.

36. Appareil protecteur d'aiguille médicale selon la revendication 35, dans lequel le dispositif de retenue comprend un bras de retenue disposé sur l'embase (18) d'aiguille et s'étendant jusqu'à un cran correspondant disposé sur le protecteur (22) dans la position rétractée.

37. Appareil protecteur d'aiguille médicale selon la revendication 35, dans lequel le dispositif de retenue comprend au moins un cran de retenue disposé sur les segments.

38. Appareil protecteur d'aiguille médicale selon la revendication 3, dans lequel le protecteur (22) comprend en outre une surface surélevée pour pousser le protecteur jusqu'à la position déployée.

39. Appareil protecteur d'aiguille médicale selon la revendication 3, dans lequel l'aiguille comporte une aiguille à extrémité ouverte.

40. Appareil protecteur d'aiguille médicale selon la revendication 3, dans lequel l'aiguille comporte une aiguille à double paroi.

41. Appareil protecteur d'aiguille médicale selon la revendication 3, dans lequel l'extrémité distale de l'aiguille comprend un biseau aligné dans un plan de symétrie avec le protecteur (22) pour indiquer l'orientation du biseau..
